(19) Europäisches Patentamt
European Patent Office
Office européen des brevets

(11) EP 0 703 441 A1

(12) EUROPÄISCHE PATENTANMELDUNG

(43) Veröffentlichungstag:
27.03.1996 Patentblatt 1996/13

(51) Int. Cl.⁶: $G01N\ 7/02$, $G01N\ 25/56$

(21) Anmeldenummer: 95110043.7

(22) Anmeldetag: 28.06.1995

(84) Benannte Vertragsstaaten:
AT BE CH DE ES FR GB IT LI NL

(30) Priorität: 20.09.1994 DE 4433451

(71) Anmelder: TESTO GmbH & Co.
D-79853 Lenzkirch (DE)

(72) Erfinder:
• Pahlke, Jörg
D-79822 Titisee-Neustadt (DE)

• Brödel, Axel, Dr.
D-79853 Lenzkirch (DE)
• Rombach, Martin
D-79853 Lenzkirch (DE)

(74) Vertreter: Patentanwälte
Westphal, Buchner, Mussgnug
Neunert, Göhring
Waldstrasse 33
D-78048 Villingen-Schwenningen (DE)

(54) **Verfahren und Vorrichtung zur Bestimmung des Wasserdampfgehaltes in Gasen**

(57) Das Verfahren zur Bestimmung des Wasserdampfgehaltes von Gasen, bei welchem ein Probengasstrom von dem zu untersuchenden Gas abgezogen wird, besteht aus folgenden vier Verfahrensschritten. In einem ersten Verfahrensschritt wird ein Volumen- und/oder Massenstrom des Probengasstromes gemessen. Anschließend wird in einem zweiten Verfahrensschritt der Wasserdampfgehalt des Probengasstroms bis zu einem bestimmten Restwasserdampfgehalt getrocknet. Nachfolgend wird in einem dritten Verfahrensschritt der Volumen- und/oder Massenstrom des Probengasstroms erneut gemessen und in einem vierten Verfahrensschritt aus der Differenz der beiden ermittelten Volumen- und/oder Masseströme der Wasserdampfgehalt des Gases bestimmt.

Fig. 1

## Beschreibung

Die Erfindung betrifft ein Verfahren bzw. eine Vorrichtung zur Bestimmung des Wasserdampfgehaltes in Gasen gemäß dem Oberbegriff des Anspruchs 1 bzw. Anspruchs 14.

Für die Bestimmung des Wasserdampfgehaltes von gasförmigen Medien sind heutzutage verschiedene Methoden bekannt. Eine der genauesten Methode besteht darin, in ein Eis/Wasser- Gemisch einen definierten Volumenstrom des zu untersuchenden Gases einzuleiten, und über die gravimetrisch bestimmte Massenzunahme den Wasserdampfgehalt zu bestimmen. Diese Methode ist einerseits sehr aufwendig und außerdem nicht für kontinuierliche Messungen geeignet.

Andere Möglichkeiten der Bestimmung des Wasserdampfgehaltes bestehen darin, die relative Feuchte oder die Taupunkttemperatur zu messen. Diese Verfahren besitzen aber den Nachteil, daß ihr Anwendungsbereich sehr beschränkt ist. Die zur Bestimmung der Taupunkttemperatur verwendeten Taupunktspiegelmeßgeräte sind teuer und können nur bis zu einer Taupunkttemperatur von etwa 70° C eingesetzt werden. Außerdem wird bei Vorhandensein von Säurebildnern im zu untersuchenden gasförmigen Medium nicht der Taupunkt des Wasserdampfs sondern der Säuretaupunkt bestimmt. Über eine Umrechnung wird dann der Wasserdampfgehalt bestimmt. Diese Umrechnung führt zu entsprechenden Fehlern.

Die relative Feuchte kann auch mit Feuchtesensoren gemessen werden. Bei Feuchtesensoren besteht aber die Gefahr der Zerstörung durch Säurefraß.

Aus der EP 0 548 842 A1 ist ein Verfahren zur Bestimmung des Wasserdampfgehaltes in Rauchgasen bekannt. Bei diesem Verfahren wird der Sauerstoffgehalt im feuchten und getrockneten Probengas mittels Zirkonoxidsensoren bestimmt und aus der Differenz der Sauerstoffwerte der Wasserdampfgehalt ermittelt. Hierbei müssen die Sensoren jedoch auf eine Arbeitstemperatur von über 350° C beheizt werden.

Bei einem weiteren Verfahren, bei dem ebenfalls der Sauerstoffgehalt gemessen wird, wird das Probengas mit Luft verdünnt, so daß die Taupunkttemperatur zu keinem Zeitpunkt unterschritten wird. Über das Verdünnungsverhältnis und den bekannten Sauerstoffgehalt der Luft und des Probengases wird der Wasserdampfgehalt bestimmt. Beide Verfahren sind insgesamt sehr aufwendig und damit fehleranfällig und außerdem relativ ungenau.

Der Erfindung liegt deshalb die Aufgabe zugrunde, ein Verfahren bzw. eine Vorrichtung der genannten Art zu schaffen, das bzw. die eine kontinuierliche Messung des Wasserdampfgehaltes eines Probengases ermöglicht, einfach durchführbar ist, eine bestimmte Genauigkeit auch bei unterschiedlichsten Bedingungen gewährleistet, kostengünstig arbeitet und eine hohe Betriebssicherheit aufweist. Das Probengas kann dabei eine bekannte oder eine noch zu bestimmende Zusammensetzung aufweisen.

Gelöst wird diese Aufgabe durch die im Anspruch 1 bzw. im Anspruch 14 angegebenen Merkmale.

Vorteilhafte Weiterbildungen sind in den Unteransprüchen angegeben.

Bei dem erfindungsgemäßen Verfahren bzw. der erfindungsgemäßen Vorrichtung zur Bestimmung des Wasserdampfgehaltes von Gasen wird im wesentlichen dem Volumen- bzw. Massenstrom vor und nach dem Trocknen des Wasserdampfgehaltes des Probenstroms gemessen. Anschließend wird unter Berücksichtigung der Restfeuchte des getrockneten Gases der Wasserdampfgehalt des Drobengases ermittelt. Die Messung erfolgt kontinuierlich und erlaubt die Bestimmung mit hinreichender Genauigkeit.

Die Trocknung des Probengasstromes und damit auch des im Probengasstrom befindlichen Wasserdampfgehaltes kann auf unterschiedlichste Weise erfolgen, beispielsweise durch Ausfrieren, Absorption, Adsorption, Permeation oder eine Kombination dieser Verfahren.

Das Ausfrieren ist in der Gasanalytik das üblichste Verfahren zur Gastrocknung. Normalerweise wird bis auf eine Taupunkttemperatur von ca. 0 bis 5 °C getrocknet.

Vorrichtungen zum Durchführen des Ausfrierens sind beispielsweise sogenannte Peltier-Kühlereinrichtungen oder die Leitung des Probengasstromes durch ein Eis-Wasser-Gemisch. Der Vorteil dieses Verfahrens liegt darin, daß nur geringe Verluste an anderen Gasbestandteilen auftreten.

Eine andere Möglichkeit zum Trocknen des Probengasstromes ist die Absorption bzw. Adsorption. Hierunter versteht man die chemische bzw. physikalische Bindung des Wasserdampfes an ein Ab- bzw. Adsorptionsmittel. Die Mittel müssen vor Erreichen des Sättigungszustandes ausgetauscht und können durch einen weiteren Verfahrensschritt regeneriert werden. Als Trocknungsmittel ist beispielsweise Silikagel geeignet. Vorzugsweise wird dieses Verfahren bei verhältnismäßig geringen Feuchten eingesetzt.

Eine weitere Möglichkeit, den Probengasstrom zu trocknen, besteht in der sogenannten Permeation. Hierunter versteht man die Verwendung einer Membran oder eines Gasleitungsmateriales, welches der Wasserdampf selektiv durchdringen kann. Die Materialien sind unterschiedlich selektiv, wodurch sich auch unterschiedliche Verluste an anderen Gasbestandteilen einstellen. Ein Beispiel für ein solches Material ist Nafion, das durch chemische Bindung, Transport und Wiederfreigabe des Wasserdampfes eine sehr schnelle und selektive Trocknung des Gasstromes erlaubt.

Das Verfahren ist für Gase bekannter und auch für zu detektierender Zusammensetzung geeignet. Es ist zur Messung von feuchten Abgasen sowie feuchter Luft in Industrie und Umweltschutz anwendbar, beispielsweise bei der Feuchtebestimmung in Prozeß- und Feuerungsabgasen in Verbindung mit Gasanalysesystemen. Insbesondere wird durch die strengen Richtlinien im Umweltbereich an ein solches Verfahren ein hoher Grad an Genauigkeit und Zuverlässigkeit gestellt.

Vorzugsweise ist es auch in Verbindung mit Detektionssystemen zur Ermittlung von Emissionen von Feuerungsanlagen und Trocknungsprozessen einsetzbar.

Nachfolgend wird das erfindungsgemäße Verfahren anhand von zwei Figuren näher erläutert werden. Es zeigen

Fig. 1    ein erstes Ausführungsbeispiel einer Vorrichtung zur Bestimmung des Wasserdampfgehaltes, und

Fig. 2    ein zweites Ausführungsbeispiel einer Vorrichtung zur Bestimmung des Wasserdampfgehaltes.

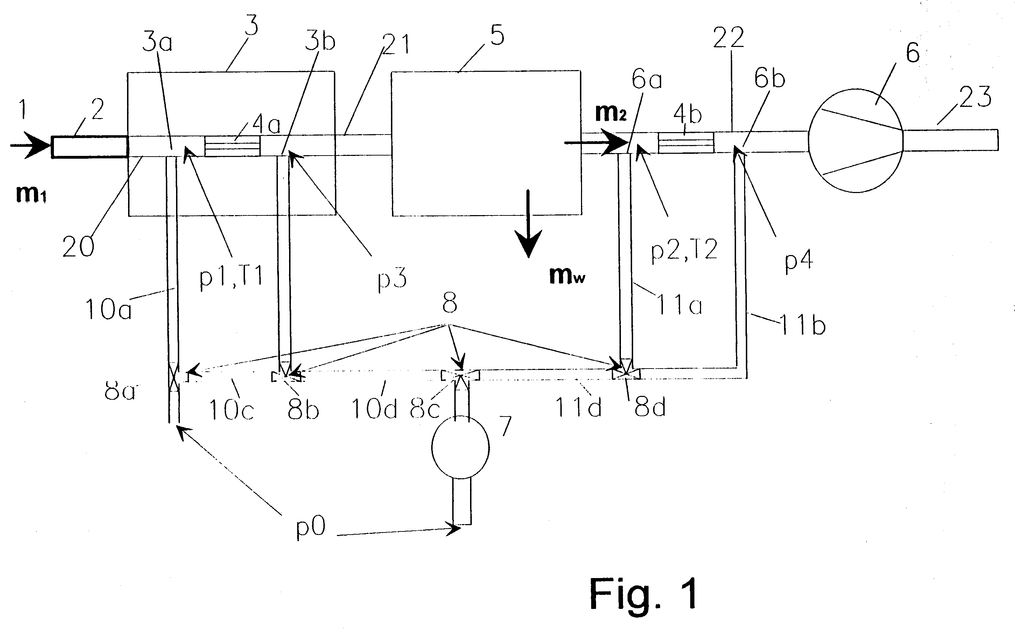

Die Figur 1 zeigt einen schematischen Aufbau einer erfindungsgemäßen Vorrichtung. Eine Ansaugvorrichtung 1 ist über einen beheizbaren Schlauch 2 mit einer Gasleitung 20 verbunden. Die Gasleitung 20 ist durch eine Kapillare 4a oder einen anderen strömungswiderstand unterbrochen. Vor und nach der Kapillare 4a zweigen jeweils eine Druckmeßleitung 10a,b an den mit 3a bzw. 3b gekennzeichneten Stellen ab. Die Gasleitung 20 mit der Kapillare 4a und die von der Gasleitung 20 anfänglich wegführenden Teile der Druckmeßleitungen 10a, b befinden sich in einer beheizbaren Kammer 3. Die aus der Kammer 3 führende Gasleitung 20 ist über eine weitere Gasleitung 21 mit einem Gaskühler 5 verbunden. Der Gaskühler 5 kann auch durch ein Eis/Wasser-Gemisch ersetzt werden. Vom Gaskühler 5 geht eine weitere Gasleitung 22 ab, die zu einer Pumpe 6 führt. Die Gasleitung 22 ist durch eine Kapillare 4b unterbrochen. Vor und nach der Kapillare 4b zweigen jeweils eine Druckmeßleitung 11a, b an den mit 6a bzw. 6b gekennzeichneten Stellen ab. Die Pumpe 6 ist ausgangsseitig mit einer Gasleitung 23 verbunden, an die beispielsweise ein Gasanalysesystem anschließbar ist.

Die Druckmeßleitungen 10a, b sind über zwei Ventile 8a, b und eine Druckmeßleitung 10c miteinander verbunden. Das Ventil 8b ist über eine Gasleitung 10d mit einem Ventil 8c verbunden. Das Ventil 8c ist mit einer Gasleitung 11d, die zu einem Ventil 8d führt, verbunden. An das Ventil 8d sind die beiden Druckmeßleitungen 11a, b angeschlossen. Das Ventil 8c ist mit einem Druckmessgerät 7 verbunden. Vom Druckmessgerät 7 sowie vom Ventil 8a führt je eine Gasleitung zur Umgebung. Bei den Ventilen 8a, b, c, d kann es sich um Zwei- oder Mehrwegventile handeln.

Die Gasleitungen 20, 21, 22, 23 sowie die angeschlossenen Druckmeßleitungen 10a, 10b, 10c, 10d und 11a, 11b, 11c, 11d und die Ventile 8a, 8b, 8c, 8d sowie der Gaskühler 5 sind gasdicht miteinander verbunden, so daß kein Gasverlust im gesamten System auftritt und das über den Schlauch 2 einströmende Gas das System über die Gasleitung 23 bis auf den auskondensierten Anteil wieder verlassen muß.

Die Kapillaren 4a, b können gleichen oder unterschiedlichen Durchmesser besitzen. Bei Verwendung anderer Strömungswiderstände können diese gleicher oder unterschiedlicher Art mit gleicher oder unterschiedlicher Größe bzw. Dimensionen sein.

Denkbar ist auch, vor oder nach dem Gaskühler 5 in die Gasleitung 21 bzw. 22 eine Lavaldüse oder einen Massenfluß-Wächter (Massflow-Controller) einzusetzen, um damit den Gasdurchsatz konstant zu halten. Dies kann auch durch die Steuerung der Pumpleistung erfolgen. Als weitere Möglichkeit, den Gasdurchsatz konstant zu halten, wäre die Geschwindigkeitsmessung an einem definierten Querschnitt des Gasleitungssystems an dem das Strömungsprofil bekannt ist, möglich. Die einzelnen Komponenten, wie Heizung für den Schlauch 2, Ventile 8a, 8b, 8c, 8d, Druckmessgerät 7, Pumpe 6 und Gaskühler 5 sind vorteilhafterweise elektrisch bzw. elektronisch steuerbar und überwachbar.

Vorteilhafter Weise werden die Meßwerte einem elektronischen Rechner zugeführt, der die Auswertung vornimmt und das ganze System steuert und überwacht.

Die in Figur 1 dargestellte Vorrichtung arbeitet folgendermaßen.

Über die Ansaugvorrichtung 1 und den beheizten Schlauch 2 wird der feuchte Probengasstrom m1 mittels der Pumpe 6 angesaugt und der beheizten Kammer 3 zugeführt. Die Temperatur T1 der Kapillare 4a an der Stelle 3a wird mit einem in der Figur 1 nicht dargestellgestellten Temperaturfühler gemessen oder gleich der Innentemperatur der Kammer 3 gesetzt. Der Schlauch 2 und die Kammer 3 können hierfür z.B. auf einer konstanten definierten Temperatur gehalten werden, die sicher über der druckabhängigen Taupunkttemperatur des feuchten Probengasstroms m1 liegt. In der Kammer 3 wird der Probengasstrom m1 durch die Kapillare 4a geführt. Vor und nach der Kapillare 4a an den mit 3a bzw. 3b gekennzeichneten Stellen wird der Systemdruck p1 und p3 gegenüber dem Umgebungsdruck p0 bestimmt. Dazu werden die Hilfsgasleitungen 10a bzw. 10b über die Ventile 8a, b, c mit dem Druckmessgerät 7 verbunden. Nach der Kammer 3 gelangt der Probengasstrom m1 über die Gasleitung 21 zum Gaskühler 5. Im Gaskühler 5 wird der Probengasstrom auf eine definierte Temperatur von z.B. ca. 5 °C abgekühlt, so daß sicher Wasserdampf auskondensiert. Dabei fällt der entsprechende Wasserdampfgehalt mw aus. Die Kühlertemperatur entspricht dann der Taupunkttemperatur des getrockneten Probengasstroms m2.

Der zumindest weitgehende trockene Probengasstrom m2 wird nach dem Gaskühler 5 über eine zweite Kapillare 4b geleitet. Hierbei werden ebenfalls die Druckwerte p2 und p4 jeweils vor und nach der Kapillare 4b an den mit 6a bzw. 6b gekennzeichneten Stellen gemessen. Hierzu werden die Druckmeßleitungen 11a bzw. 11b über die Ventile 8d und 8c mit dem Druckmessgerät 7 verbunden. Außerdem wird an der mit 6a gekennzeichneten Stelle die Temperatur T2 mittels eines in der Figur nicht dargestellten Temperaturfühlers gemessen. Die Druckdifferenzen dp1 = p3-p1 und dp2 = p4- p2 an den Kapillaren 4a bzw. 4b sind unter Beachtung der Dichte des Probengasstromes ein Maß für den Volumen- bzw. Massenstrom. Über die Bilanzgleichung

m1=m2+mw kann der Wasserdampfanteil bestimmt werden.

Der Restfeuchtegehalt des trockenen Probengasstroms kann erfindungsgemäß bestimmt oder bei nur geringen Anteilen vernachlässigt bzw. geschätzt werden. Die Bestimmung kann durch Messung der relativen Feuchte, des Taupunktes oder bei Verwendung eines Gaskühlers über die Kühlertemperatur erfolgen.

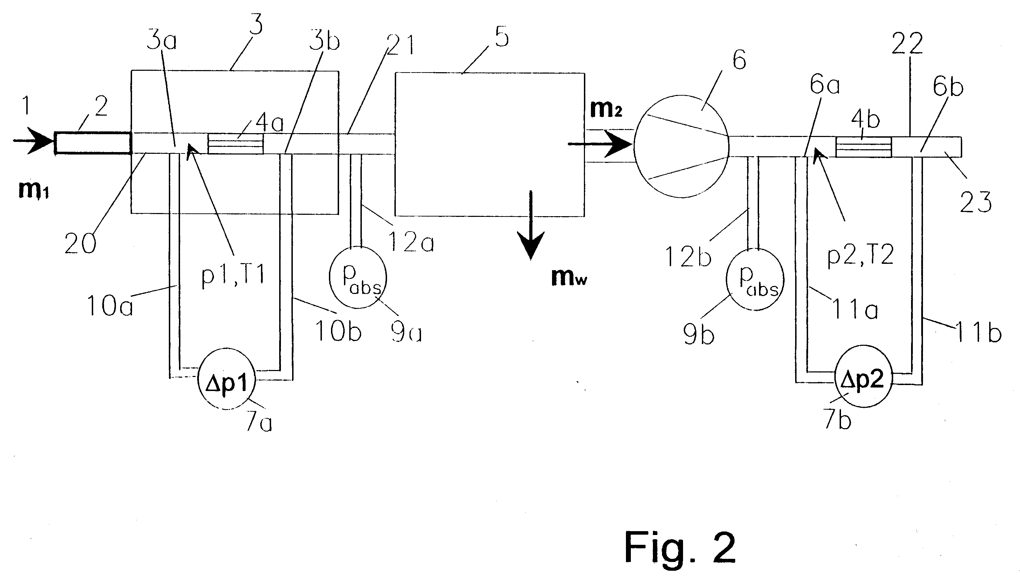

In Fig. 2 ist ein zweites Ausführungsbeispiel einer Vorrichtung zur Bestimmung des Wasserdampfgehaltes von Gasen dargestellt. Gleiche Bezugszeichen stehen wieder, sofern nicht anders angegeben, für die bereits bekannten Teile mit gleicher Bedeutung.

Im Gegensatz zu der Ausführungsform in Fig. 1 sind die Druckmeßleitungen 10a, 10b bzw. 11a, 11b an ehren den Kapillaren 4a, 4b bzw. den entsprechenden Strömungswiderständen abgewandten Enden jeweils mit einer Druckdifferenzeinrichtung 7a, 7b, z.B. einem Druckdifferenzsensor, in Verbindung. Mittels dieser Druckdifferenzmeßeinrichtung 7a bzw. 7b sind die Differenzdrücke, die zwischen dem Eingang und Ausgang der beiden Kapillaren 4a bzw. 4b herrschen, bestimmbar. Die Druckdifferenzmeßeinrichtung 7a bestimmt die Druckdifferenz dp1 und die Druckdifferenzmeßeinrichtung 7b die Druckdifferenz dp2. Zusätzlich weist die in Fig. 2 dargestellte Vorrichtung zwei weitere Absolutdruckmeßeinrichtungen 9a und 9b, beispielsweise Absolutdrucksensoren, auf. Die Absolutdruckmeßeinrichtung 9a ist über eine weitere Druckmeßleitung 12a mit der Gasleitung 21 vor dem Gaskühler 5 in Verbindung. Die Absolutdrukmeßeinrichtung 9b ist dagegen mit einer weiteren Druckmeßleitung 12b mit der Gasleitung 22 in Verbindung, und zwar im Bereich der eingangsseitigen Stelle 6a der Kapillare 4b.

In Unterschied zur Darstellung von Fig. 1 ist die Pumpe 6 jetzt nicht ausgangsseitig an die Kapillare 4b, sondern zwischen die Kapillare 4b und den Gaskühler 5 geschaltet. Die Absolutdruckmeßeinrichtung 9b liegt zwischen der Ausgangsseite dieser Pumpe 6 und der Kapillare 4b. Es ist hier noch zu erwähnen, daß nicht notwendigerweise ein Gaskühler 5 in den in Fig. 1 und 2 vorgestellten Vorrichtungen verwendet werden muß. Es ist vielmehr jede Einrichtung hier verwendbar, die den Wasserdampfgehalt des Probengasstromes austrocknet oder zumindest weitgehend austrocknen kann. Als mögliche Verfahren des Austrocknens kommen Ausfrieren, Absorption, Adsorption, Permeation oder eine Kombination dieser Verfahren in Betracht.

Das erfindungsgemäße Verfahren ist für Probengasströme bekannter und unbekannter Zusammensetzung anwendbar. Der Probengasstrom kann aus einem Medien bekannter chemischer Zusammensetzung, z.B. Luft, bestehen. Besteht der Probengasstrom aus einem Medium, dessen chemische Zusammensetzung unbekannt ist, so besteht die Möglichkeit, diese Zusammensetzung durch ein Gasanalysesystem quantitativ zu bestimmen z.B. wenn es sich um Abgase aus Verbrennungsprozessen handelt, deren Zusammensetzung in der Regel nur qualitativ bekannt ist. Eine weitere Möglichkeit, die Gasdichte des Probengasstromes zu bestimmen, besteht darin, diese gravimetrisch oder durch Vergleich der Ausstömgeschwindigkeiten zu ermitteln.

Die wesentlichen Merkmale des Verfahrens bestehen darin, den Volumenstrom des feuchten Probegasstromes m1 zu messen. Dies muß bei einer Temperatur, die höher als die Taupunkttemperatur ist, erfolgen. Druck und Temperatur des Probengasstromes m1 sind zu messen oder müssen bekannt sein. Anschließend erfolgt ein Auskondensieren des Wasserdampfgehaltes des Probengasstromes m1 bis auf einen Restgehalt. Dieser Restgehalt ist über Druck- und Temperaturwerte konstant zu halten oder bestimmbar. Eine weitere Möglichkeit, diesen Restgehalt zu bestimmen, besteht in einer Feuchte- oder Taupunktmessung. Nach der Auskondensation erfolgt eine Bestimmung des Volumenstromes des trockenen Probengasstromes m2. Druck und Temperatur des Probengasstromes m2 sind zu messen oder müssen konstant sein.

Die Bestimmung der Normdichte des trockenen Probengasstromes m2 erfolgt entweder durch die Ermittlung der chemischen Zusammensetzung oder durch Messen der Gasdichte. Der Wasserdampfgehalt kann dann aus den Meßwerten ermittelt werden.

**Patentansprüche**

1. Verfahren zur Bestimmung des Wasserdampfgehaltes von Gasen, bei welchem ein Probengasstrom von dem zu untersuchenden Gas abgezogen wird, dadurch gekennzeichnet, daß in einem ersten Verfahrensschritt ein Volumen- und/oder Massenstrom (m1) des Probengasstromes gemessen wird, daß in einem zweiten Verfahrensschritt der Wasserdampfgehalt des Probengasstroms bis zu einem bestimmten Restwasserdampfgehalt getrocknet wird, daß in einem nachfolgenden dritten Verfahrensschritt der Volumen- und/oder Massenstrom (m2) des Probengasstroms erneut gemessen wird, und daß in einem vierten Verfahrensschritt aus der Differenz der beiden ermittelten Volumen- und/oder Masseströme (m1, m2) der Wasserdampfgehalt des Gases bestimmt wird.

2. Verfahren nach Anspruch 1, dadurch gekennzeichnet, daß die Volumen- bzw. Massenströme (m1 bzw. m2) aus Druckdifferenzen (dp1 bzw. dp2), die vor und nach einem Strömungswiderstand (4a bzw. 4b), durch welche der Probengasstrom geführt wird, bei einer Temperatur (T1 bzw. T2) auftreten, bestimmt werden.

3. Verfahren nach Anspruch 1, dadurch gekennzeichnet, daß ein Volumen- oder Massenstrom (m1 bzw. m2) des feuchten bzw. trockenen Probengasstroms konstant gehalten wird.

4. Verfahren nach Anspruch 3, dadurch gekennzeichnet, daß zur Aufrechterhaltung des konstanten Volumen- oder Massenstroms (m1 bzw. m2) eine Lavaldüse oder ein Massenflußwächter (Massflow-Contoller) verwendet wird.

5. Verfahren nach einem der vorhergehenden Ansprüche, dadurch gekennzeichnet, daß der Volumen- oder Massenstrom (m1 bzw. m2), durch die Steuerung der Pumpleistung der Pumpe 6 gesteuert bzw. geregelt wird.

6. Verfahren nach einem der vorhergehenden Ansprüche, dadurch gekennzeichnet, daß der Volumen- oder Massenstrom (m1 bzw. m2) des Probengasstroms durch Geschwindigkeitsmessung in einem definierten Querschnitt bekannten Strömungsprofils erfolgt.

7. Verfahren nach Anspruch 1, dadurch gekennzeichnet, daß die Trocknung des Probengasstroms durch Kühlung auf eine konstante Temperatur erfolgt.

8. Verfahren nach Anspruch 7, dadurch gekennzeichnet, daß die Kühlung des Probengasstroms auf eine konstante Temperatur durch Einleiten des Probengasstroms in ein Eis/Wasser-Gemisch oder in einen Gaskühler (5) erfolgt.

9. Verfahren nach Anspruch 8, dadurch gekennzeichnet, daß der Restfeuchtegehalt des Probengasstroms nach der Kondensation über die Kühltemperatur bestimmt wird.

10. Verfahren nach Anspruch 1, dadurch gekennzeichnet, daß die Restfeuchte des getrockneten Probengasstromes durch ein vorgegebenes Trocknungsmittel, insbesondere Silikagel, so weit reduziert wird, daß sie für die Bestimmung des Wasserdampfgehaltes vernachlässigbar oder über einen Schätzwert berücksichtigt wird.

11. Verfahren nach einem der Ansprüche 1 bis 10, dadurch gekennzeichnet, daß die Trocknung des Probengasstromes durch Ausfrieren, Absorption, Adsorption, Permeation oder Kombination dieser Verfahren erfolgt.

12. Verfahren nach Anspruch 1, dadurch gekennzeichnet, daß der Restfeuchtegehalt des getrockneten Probengasstroms durch eine Messung der relativen Feuchte oder des Taupunkts bestimmt wird.

13. Verfahren nach Anspruch 1, dadurch gekennzeichnet, daß der Probengasstrom aus einem feuchten gasförmigen Medium bekannter chemischer Zusammensetzung, z.B. Luft, besteht.

14. Verfahren nach Anspruch 1, dadurch gekennzeichnet, daß der Probengasstrom in seiner qualitativen chemischen Zusammensetzung bekannt ist und die wesentlichen Bestandteile durch Detektion mittels eines Gasanalysesystems und Berechnung qualitativ bestimmt werden.

15. Verfahren nach Anspruch 1, dadurch gekennzeichnet, daß der trockene Probengasstrom in seiner chemischen Zusammensetzung unbekannt ist und die Gasdichte desselben gravimetrisch oder durch Vergleich von Ausströmgeschwindigkeiten ermittelt wird.

16. Vorrichtung zur Bestimmung des Wasserdampfgehaltes von Gasen mit einer Gaszuleitung (20), die zu einer Trocknungsvorrichtung (5) führt und einer Gasableitung (22), die von dieser wegfuhrt, dadurch gekennzeichnet, daß die Gaszuleitung (20) und die Gasableitung (22) jeweils eine Vorrichtung zur Bestimmung des Volumen- bzw. Massenstromes (m1 bzw. m2) eines in der Gaszuleitung (20) bzw. Gasableitung (22) geführten Probengasstromes aufweisen, und daß eine Auswerteeinrichtung vorgesehen ist, um aus der Differenz der beiden ermittelbaren Volumen- bzw. Masseströme (m1 bzw. m2) den Wasserdampfgehalt des Gases zu bestimmen.

17. Vorrichtung nach Anspruch 16, dadurch gekennzeichnet, daß die Vorrichtung zur Bestimmung des Volumen- bzw. Massenstromes aus einem definierten Strömungswiderstand (4a bzw. 4b) besteht, dessen Eingänge (3a bzw. 6a) und Ausgänge (3b bzw. 6b) jeweils mit einem Druckmessgerät (7) verbindbar sind.

18. Vorrichtung nach Anspruch 16, dadurch gekennzeichnet, daß in der Zu- oder Ableitung des Gasweges (22) eine Pumpe (6) angeordnet ist.

19. Vorrichtung nach Anspruch 16, dadurch gekennzeichnet, daß mittels einer Lavaldüse oder eines Massflow-Controllers ein Volumen- oder Massenstrom (m1 bzw. m2) konstant gehalten wird.

20. Vorrichtung nach Anspruch 16, dadurch gekennzeichnet, daß durch eine Absaugvorrichtung auf Seite der Gasableitung ein Unterdruck erzeugt und über einen Strömungswiderstand ein Stellglied vorgesehen ist.

Fig. 1

EP 0 703 441 A1

Fig. 2

EP 0 703 441 A1

Europäisches
Patentamt

**EUROPÄISCHER RECHERCHENBERICHT**

Nummer der Anmeldung

EP 95 11 0043

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | US-A-4 724 700 (JAASMA DENNIS R) 16.Februar 1988 <br><br> * Spalte 2, Zeile 45 - Spalte 3, Zeile 62; Abbildung 1 * <br> --- | 1-3,5,7, 8,10,11, 14,16-18 | G01N7/02 G01N25/56 |
| X | US-A-4 507 875 (HIRSCH WERNER ET AL) 2.April 1985 <br> * Spalte 3, Zeile 39 - Spalte 4, Zeile 47 * <br> * Spalte 5, Zeile 29 - Zeile 47; Abbildungen 2,3 * <br> --- | 1,3,5-8, 16,18 | |
| X | US-A-5 050 109 (LADD MICHAEL M) 17.September 1991 <br> * Spalte 1, Zeile 43 - Spalte 2, Zeile 33; Abbildung 1 * <br> --- | 1,9,12, 13,15,16 | |
| A | US-A-5 187 972 (DEFRIEZ HERBERT H) 23.Februar 1993 <br><br> * Spalte 4, Zeile 4 - Spalte 8, Zeile 45; Abbildungen 1-6 * <br> --- | 1-3,5,7, 10,11, 13,16-18 | **RECHERCHIERTE SACHGEBIETE (Int.Cl.6)** <br><br> G01N |
| D,A | EP-A-0 548 842 (SAARBERGWERKE AG ;YOKOGAWA DEUTSCHLAND GMBH (DE)) 30.Juni 1993 <br> ----- | | |

Der vorliegende Recberchenbericht wurde für alle Patentansprüche erstellt

| Recherchenort | Abschlußdatum der Recherche | Prüfer |
|---|---|---|
| DEN HAAG | 1.Dezember 1995 | Hodson, M |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus andern Gründen angeführtes Dokument
............................................................
& : Mitglied der gleichen Patentfamilie, übereinstimmendes
  Dokument

EPO FORM 1503 03.82 (P04C03)